# EUROPÄISCHE PATENTSCHRIFT

(11) **EP 4 444 685 B1**
(45) Veröffentlichungstag und Bekanntmachung des Hinweises auf die Patenterteilung: **23.07.2025**
(21) Anmeldenummer: 22839144.7
(22) Anmeldetag: 08.12.2022
(51) Int. Cl.: C07C 5/48, C07C 7/00, C07C 7/04, C07C 7/167, C07C 51/25, C07C 11/04, C07C 9/06, C07C 53/08, C10G 9/36, C10G 51/06, C10G 70/00, C10G 70/02, C10G 70/04

(54) **VERFAHREN UND ANLAGE ZUR HERSTELLUNG EINES ODER MEHRERER KOHLENWASSERSTOFFE**
METHOD AND INSTALLATION FOR THE PRODUCTION OF ONE OR MORE HYDROCARBONS
PROCÉDÉ ET INSTALLATION DE PRODUCTION D'AU MOINS UN HYDROCARBURE

(30) Priorität: 08.12.2021 EP 21020622
(43) Veröffentlichungstag der Anmeldung: 16.10.2024
(73) Patentinhaber: Linde GmbH, 82049 Pullach (DE)
(72) Erfinder: KRACKER, Gunther, 83629 Weyarn (DE); ZELLHUBER, Mathieu, 82152 Planegg (DE); MCCRACKEN, Sean, 82178 Puchheim (DE); MEISWINKEL, Andreas, 83253 Rimsting (DE); SCHUBERT, Martin, 81375 München (DE); TOTA, Desislava, 81479 München (DE)
(86) Internationale Anmeldenummer: PCT/EP2022/084975
(87) Internationale Veröffentlichungsnummer: WO 2023/104962

(56) Entgegenhaltungen:
- WO-A1-2018/024650

## Beschreibung

Die Erfindung betrifft ein Verfahren und eine Anlage zur Herstellung eines oder mehrerer Kohlenwasserstoffe.

### Technischer Hintergrund

Das Steamcracken von Kohlenwasserstoffen (engl. Steam Cracking, im Deutschen auch als Dampfspalten bezeichnet) ist beispielsweise im Artikel "Ethylene" in Ullmann's Encyclopedia of Industrial Chemistry, Onlineausgabe, 15. April 2009, DOI: 10.1002/14356007.a10_045.pub2, beschrieben. Es wird vorwiegend zur Gewinnung von kurzkettigen Olefinen wie Ethylen und Propylen, Diolefinen wie Butadien, oder von Aromaten eingesetzt, ist jedoch nicht hierauf beschränkt.

Die oxidative Dehydrierung (engl. Oxidative Dehydrogenation, ODH) von Paraffinen mit zwei bis vier Kohlenstoffatomen (im Falle der oxidativen Dehydrierung von Ethan zu Ethylen auch als ODHE bzw. ODH-E bezeichnet) ist ebenfalls bekannt. Bei der oxidativen Dehydrierung werden die genannten Paraffine mit Sauerstoff unter anderem zu den jeweiligen Olefinen und zu Wasser umgesetzt. Sie stellt ein alternatives und in bestimmten Fällen vorteilhaftes Verfahren zur Herstellung von Olefinen dar.

Wie nachfolgend noch erläutert, sind beispielsweise aus der WO 2018/024650 A1, der WO 2014/134703 A1 und der CN 103086824 B auch Kombinationsverfahren aus Steamcracken und oxidativer Dehydrierung bekannt. Die vorliegende Erfindung stellt sich die Aufgabe, entsprechende Kombinationsverfahren zu verbessern.

### Offenbarung der Erfindung

Es wird ein Verfahren zur Herstellung eines oder mehrerer Kohlenwasserstoffe vorgeschlagen, bei dem ein erster Einsatzstrom unter Erhalt eines ersten Produktstroms einem Steamcracken und ein Ethan enthaltender zweiter Einsatzstrom unter Erhalt eines zweiten Produktstroms einer oxidativen Dehydrierung unterworfen werden, wobei zumindest ein Teil des ersten Produktstroms unter Erhalt von Kohlenwasserstofffraktionen einer Aufbereitung unterworfen wird, die eine Selektivhydrierung von Kohlenwasserstoffen mit zwei Kohlenstoffatomen und eine Demethanisierung umfasst, wobei zumindest ein Teil des zweiten Produktstroms unter Erhalt eines Folgestroms einer Spurenentfernung unterworfen wird, die eine Entfernung von Sauerstoff und/oder Acetylen umfasst, und wobei zumindest ein Teil des Folgestroms der Aufbereitung an einer Einspeisestelle stromab der Selektivhydrierung und stromauf der Demethanisierung zugeführt wird. Zumindest ein Teil des Folgestroms wird stromauf der Einspeisestelle in die Aufbereitung einer Entfernung von Kohlendioxid unterworfen. Diese im Rahmen der vorliegenden Erfindung erfolgende separate Entfernung von Kohlendioxid aus dem zweiten Produktstrom ist besonders vorteilhaft, weil sie nicht zu Lasten der ggf. vorhandenen, kapazitätslimitierten Entfernung stromab des Steamcrackers geht.

Ein Restsauerstoffgehalt des Folgestroms beträgt in dem vorgeschlagenen Verfahren weniger als 500 vol.-ppm und ein Restacetylengehalt des Folgestroms beträgt im Rahmen des vorgeschlagenen Verfahrens weniger als 5 vol.-ppm.

Ferner wird hierdurch, wie unten noch erläutert, der Laugeverbrauch reduziert bzw. gering gehalten, insbesondere dann, wenn eine Kohlendioxid-Feinreinigung des ersten Produktstroms mittels Laugewäsche vorgenommen wird. Dies ist insbesondere deshalb der Fall, weil der erste Produktstrom, entsprechend der Herkunft aus dem Steamcracker, vergleichsweise wenig Kohlendioxid aufweist und daher eine Entfernung von Kohlendioxid in diesem ersten Produktstrom bereits nur durch eine Laugewäsche technisch und wirtschaftlich sinnvoll erreicht werden kann. Eine vorherige Aminwäsche ist daher nicht erforderlich und in bestehenden Steamcrackern üblicherweise nicht vorhanden. Würde eine derartige Laugewäsche nun auch für die Entfernung der größeren Kohlendioxidmengen aus dem zweiten Produktstrom verwendet, müsste ein entsprechender Anlagenteil sehr stark erweitert werden bzw. es ergäbe sich ein hoher Laugeverbrauch. Dies wird durch die hier vorgeschlagenen Maßnahmen sicher verhindert.

Lediglich zur Klarstellung sei nochmals festgehalten, dass in allen Ausgestaltungen der Erfindung zumindest ein Teil des ersten Produktstrom stromauf der Einspeisestelle des Folgestroms in die Aufbereitung einer Entfernung von Kohlendioxid unterworfen wird, die insbesondere eine Laugewäsche, und in bestimmten Ausgestaltungen der Erfindung ausschließlich eine Laugewäsche, umfassen kann.

Gemäß einer Ausgestaltung der Erfindung umfasst die Entfernung von Kohlendioxid aus dem Folgestrom oder einem Teil hiervon zumindest eine regenerative Wäsche, insbesondere Aminwäsche und ggf. optional eine Feinreinigung per Laugewäsche. Auf diese Weise kann insbesondere ein geringer Restgehalt an Kohlendioxid in besonders vorteilhafter Weise erzielt werden. Ein solcher geringer Restgehalt an Kohlendioxid entspricht insbesondere größenordnungsmäßig dem Restgehalt, wie er in der Kohlendioxidentfernung aus dem ersten Produktstrom erreicht wird.

Wie auch nachfolgend erläutert, wird durch die vorliegende Erfindung eine optimierte Integration von Steamcracken und oxidativer Dehydrierung geschaffen, die eine gemeinsame Nutzung insbesondere eines Demethanizers und einer Trenneinheit zur Trennung von Kohlenwasserstoffen mit zwei Kohlenstoffatomen (C2-Splitter) erlaubt. Die Spurenentfernung ermöglicht, dabei eine druck- bzw. explosionsfeste Auslegung von Anlagenteilen zu vermeiden. Es ist insbesondere eine bedarfsgerechte Optimierung und Anpassung der Ethylenkapazität möglich.

Gemäß einer Ausgestaltung der Erfindung erfolgt die oxidative Dehydrierung unter Verwendung eines oder mehrerer, die Metalle Molybdän, Vanadium, Niob und optional Tellur enthaltender Katalysatoren. Entsprechende Katalysatorsysteme sind, wie auch nachfolgend noch erläutert, bewährt und robust und erlauben neben einer vorteilhaften Gewinnung von Olefinen auch ggf. die Herstellung von entsprechenden organischen Säuren in Koppelproduktion.

Gemäß einer Ausgestaltung der Erfindung umfasst die Aufbereitung eine Deethanisierung und/oder eine Depropanisierung, wobei die Selektivhydrierung stromab der Deethanisierung und/oder stromab der Depropanisierung vorgenommen wird. Der besondere Vorteil einer entsprechenden Anordnung besteht dabei darin, dass insbesondere Prozessschritte wie eine Verdichtung, Laugewäsche und Selektivhydrierung im Trennteil einer Steamcrackeranlage oftmals kapazitätslimitierend bei einer Nachrüstung bzw. Kapazitätserweiterung sind. Diese Prozessschritte sind durch eine Integration gemäß einer Ausgestaltung der Erfindung vorteilhafterweise jedoch nicht betroffen. Die hingegen betroffenen Prozessschritte, insbesondere Trennungen wie Deethanisierung, Demethanisierung und Trennung von Kohlenwasserstoffen mit zwei Kohlenstoffatomen bieten dagegen, wie hier erkannt, oftmals Reserven bzw. lassen sich leichter in Ihrer Kapazität erweitern.

Gemäß einer Ausgestaltung der Erfindung umfasst die Aufbereitung eine Deethanisierung, wobei die Selektivhydrierung stromauf der Deethanisierung vorgenommen wird. Durch eine derartige Ausgestaltung wird insbesondere die sich anschließende Abtrennung von Kohlenwasserstoffen mit zwei Kohlenstoffatomen und deren nachfolgende Trennung voneinander vereinfacht.

Gemäß einer Ausgestaltung der Erfindung wird zumindest ein Teil des Folgestroms stromauf der Einspeisestelle in die Aufbereitung (neben der Entfernung von Kohlendioxid) einer Verdichtung und/oder einer Trocknung unterworfen. Auf diese Weise werden auch diese, ggf. kapazitätsbegrenzenden Schritte in der Aufbereitung des Produktgemischs aus dem Steamcracken nicht belastet.

Gemäß einer Ausgestaltung der Erfindung wird in der Selektivhydrierung ein zumindest Palladium enthaltender Katalysator verwendet. Besonders vorteilhafte und technisch übliche Katalysatoren basieren auf Palladium, können aber auch noch mit anderen Elementen dotiert sein, insbesondere Silber, Gold, Cer u.a., um die katalytischen Eigenschaften weiter zu verbessern. Entsprechende Katalysatoren sind typischerweise sehr sensitiv auf den Gehalt an Kohlenmonoxid im Prozessgas.

Gemäß einer Ausgestaltung der Erfindung wird bzw. werden in der Spurenentfernung ein oder mehrere, Kupferoxid enthaltende Katalysatoren oder ein oder mehrere, zumindest eines der Elemente Kupfer, Mangan, Zink, Nickel, Platin, Palladium, Rhodium und/oder Ruthenium enthaltende Katalysatoren verwendet. Merkmale und Vorteile sind beispielsweise in der WO 2020/187572 A1 diskutiert, auf welche daher hier ausdrücklich Bezug genommen wird.

Gemäß einer Ausgestaltung der Erfindung beträgt ein Restsauerstoffgehalt des Folgestroms weniger als 250 vol.-ppm, 100 vol.-ppm, 10 vol.-ppm oder 1 vol.-ppm. Insbesondere ist ein höherer Sauerstoffgehalt möglich als typischerweise im Rohgasstrom eines Steamcrackers, da nach Zuführung an der Einspeisestelle keine Diene mehr vorhanden sind und kein Fouling erwartet wird. Dennoch ist der Wert ausreichend niedrig, um eine sicherheitstechnische Gefährdung durch Anreicherung in einer leichten Fraktion aus Methan und anderen leicht siedenden Komponenten zu vermeiden, so dass sich sicherheitstechnische Vorteile ergeben. Weil Sauerstoff nicht extrem tief entfernt werden muss und ein verbleibender Restgehalt in einer Demethanisierung entfernt wird, ergibt sich insbesondere eine Minimierung des Ethylenverlustes in einer Rohgasbehandlung. Details sind weiter unten erläutert.

Generell soll hier unter dem Begriff "Rohgasbehandlung" eine Behandlung des Produktstroms aus der oxidativen Dehydrierung, oder eines hiervon abgeleiteten Stroms, verstanden werden, unabhängig davon, an welcher Stelle eines entsprechenden Prozesses sie stattfindet. Eine Rohgasbehandlung im hier verstandenen Sinn erfolgt jedoch immer vor der Vereinigung mit einem Strom aus dem Steamcracken.

Gemäß einer Ausgestaltung der Erfindung wird in der Aufbereitung stromab der Einspeisestelle eine Abkühlung zumindest eines Teils des jeweils bearbeiteten Gasgemischs auf Temperaturen von weniger als -120°C, -135°C oder -150°C vorgenommen. Insbesondere ist eine Nutzung von Spitzenkälte in einer Demethanisierung möglich, durch welche sich eine Reduzierung der Ethylenverluste in der leichten Fraktion aus der Demethanisierung ergibt.

Gemäß einer Ausgestaltung der Erfindung beträgt ein Restacetylengehalt des Folgestroms weniger als 2 vol.-ppm, 1 vol.-ppm, 0,5 vol.-ppm, 0,3 vol.-ppm oder 0,1 vol.-ppm. Einerseits sind entsprechende Werte besonders relevant für eine Einhaltung der Ethylenspezifikation und andererseits liefert die Rohgasbehandlung stromab einer oxidativen Dehydrierung ggf. besonders geringe Acetylengehalte.

Gemäß einer Ausgestaltung der Erfindung umfasst die Aufbereitung eine Trennung von Kohlenwasserstoffen mit zwei Kohlenstoffatomen, in der ein an Ethan angereicherter Strom gewonnen wird, wobei der an Ethan angereicherte Strom zumindest teilweise zum Steamcracken und/oder zur oxidativen Dehydrierung als Teil des ersten und/oder zweiten Einsatzstroms zurückgeführt wird. Mit einer derartigen Ausgestaltung ist insbesondere eine Kapazitätserweiterung eines Steamcrackers durch Nutzung der oxidativen Dehydrierung insbesondere für einen entsprechenden Ethanrecycle möglich.

Gemäß einer Ausgestaltung der Erfindung erfolgt die Spurenentfernung mittels einer Rohgasbehandlung, die vor oder nach einer Verdichtung angeordnet ist.

Gemäß einer Ausgestaltung der Erfindung wird zumindest ein Teil des zweiten Produktstroms einer Kondensatabtrennung unterworfen, in der ein Kondensatstrom abgetrennt wird, der mindestens 1 Gew.-% Essigsäure enthält, wobei der Kondensatstrom insbesondere einer weiteren Aufbereitung unterzogen wird, um Essigsäure als Wertprodukt zu gewinnen. Die Erfindung ermöglicht in einer entsprechenden Ausgestaltung eine vorteilhafte Gewinnung dieses Wertprodukts.

Eine Anlage, die zur Durchführung eines Verfahrens in einer beliebigen Ausgestaltung der Erfindung eingerichtet ist, ist ebenfalls Gegenstand der Erfindung und profitiert in gleicher Weise von den vorstehend zu einzelnen Ausgestaltungen erwähnten und nachfolgend noch erläuterten Vorteilen.

### Kurze Beschreibung der Zeichnungen

Figur 1 veranschaulicht ein Verfahren gemäß einer Ausgestaltung der Erfindung.
Figur 2 veranschaulicht ein Verfahren gemäß einer Ausgestaltung der Erfindung.

### Ausführliche Beschreibung

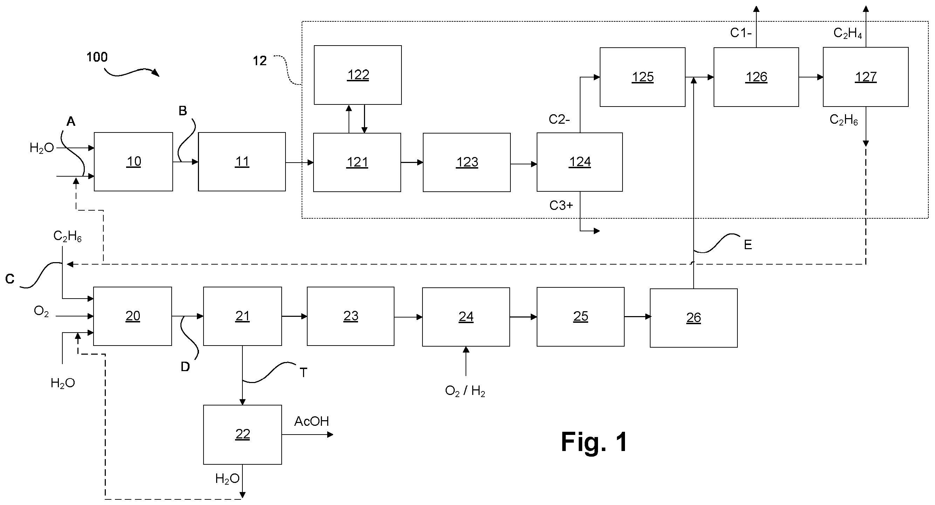

In Figur 1 ist ein Verfahren gemäß einer Ausgestaltung der Erfindung in Form eines schematischen Ablaufplans veranschaulicht und insgesamt mit 100 bezeichnet.

Als zentrale Verfahrensschritte sind hier ein Steamcracken 10 unter Verwendung eines oder mehrerer Prozesseinheiten wie Spaltöfen und eine oxidative Dehydrierung 20, die unter Verwendung eines oder mehrerer Reaktoren durchgeführt werden kann, veranschaulicht. Dem Steamcracken 10 ist ein beispielsweise fachüblicher Quenchschritt 11 nachgeschaltet. Ihm schließt sich eine insgesamt mit 12 angegebene Aufbereitung an. Die der oxidativen Dehydrierung 20 nachgeschalteten Schritte sind weiter unten beschrieben.

Die Aufbereitung 12 umfasst eine Verdichtung 121, eine (üblicherweise) auf einer Zwischenstufe der Verdichtung 121 vorgenommene Entfernung 122 von Kohlendioxid, eine Trocknung 123, eine Deethanisierung 124 unter Gewinnung einer schwereren Fraktion C3+ mit Kohlenwasserstoffen mit drei Kohlenstoffatomen und schwereren Kohlenwasserstoffen und einer leichteren Fraktion C2- mit Kohlenwasserstoffen mit zwei Kohlenstoffatomen, Methan, Kohlenmonoxid und ggf. leicht siedenden Komponenten (wie verbleibendem Sauerstoff), eine Selektivhydrierung 125 von Kohlenwasserstoffen mit zwei Kohlenstoffatomen, eine Demethanisierung 126 unter Abtrennung einer leichten Fraktion C1- mit Methan und anderen leicht siedenden Komponenten und eine Trennung 127 von Kohlenwasserstoffen mit zwei Kohlenstoffatomen voneinander unter Erhalt einer Ethylenfraktion C₂H₄ und einer Ethanfraktion C₂H₆. Letztere kann, wie in Form eines gestrichelten Pfeils veranschaulicht, in das Steamcracken 10 und/oder in die oxidative Dehydrierung 20 zurückgeführt werden.

Der oxidativen Dehydrierung 20 ist eine Abtrennung 21 von Kondensat nachgeschaltet, in der ein Kondensatstrom T gewonnen wird. Dieser wird einer Kondensataufbereitung 22 zugeführt, in der eine Essigsäurefraktion AcOH und eine Wasserfraktion H₂O gewonnen werden können. Letztere kann, wie in Form eines gestrichelten Pfeils veranschaulicht, in das Steamcracken 10 und/oder in die oxidative Dehydrierung 20 zurückgeführt werden. Der Abtrennung 21 von Kondensat ist eine Verdichtung 23 nachgeschaltet, stromab derer eine Spurenentfernung 24 vorgenommen wird, die eine Entfernung von Sauerstoff und/oder Acetylen umfasst, und der optional Sauerstoff O₂, Kohlenmonoxid CO und/oder Wasserstoff H₂ zugeführt werden können. Eine Entfernung 25 von Kohlendioxid und optional eine Trocknung 26 schließen sich an. Eine Spurenentfernung 24 kann alternativ auch vor einer Verdichtung 23 oder auf einer Zwischenstufe der Verdichtung 23 erfolgen.

Wie hier veranschaulicht, wird dem Steamcracken 10 ein hier mit A bezeichneter erster Einsatzstrom zugeführt und dieser dort unter Erhalt eines hier mit B bezeichneten ersten Produktstroms bearbeitet. Dem Steamcracken 10 wird ferner Wasser H₂O in Form von Dampf zugeführt. Ein hier mit C bezeichneter, Ethan C₂H₆ enthaltender zweiter Einsatzstrom wird unter Erhalt eines hier mit D bezeichneten zweiten Produktstroms der oxidativen Dehydrierung 20 unterworfen. Der oxidativen

Dehydrierung 20 werden ferner Wasser H₂O in Form von Dampf und Sauerstoff O₂ zugeführt. Zumindest ein Teil des ersten Produktstrom B wird unter Erhalt (zumindest) der Kohlenwasserstofffraktionen (C₂H₄, C₂H₆) der Aufbereitung 12 unterworfen, die die erwähnte Selektivhydrierung 125 von Kohlenwasserstoffen mit zwei Kohlenstoffatomen und die ebenfalls erwähnte Demethanisierung 126 umfasst. Zumindest ein Teil des zweiten Produktstroms D (hier der nach der Kondensatabtrennung verbleibende Teil) wird unter Erhalt eines hier mit E bezeichneten Folgestroms E (zumindest) der Spurenentfernung 24 unterworfen, die eine Entfernung von Sauerstoff und/oder Acetylen umfasst. Zumindest ein Teil (hier der nach Kohlendioxidentfernung 25 und Trocknung 26 verbleibende Rest des Folgestroms E) wird dann der bereits oben diskutierten Aufbereitung 12 an einer Einspeisestelle stromab der Selektivhydrierung 125 und stromauf der Demethanisierung 126 zugeführt. Die Selektivhydrierung 125 ist hier als Frontend-Hydrierung ausgeführt. Die Führung der weiteren Stoffströme, die nicht gesondert bezeichnet sind, ergibt sich unmittelbar aus der Darstellung. In Ausgestaltungen der Erfindung können Prozessschritten auch in geeigneter Weise vertauscht sein, z.B. in Form eines an sich bekannten "Depropanizer First"-Verfahrens mit anschließender Selektivhydrierung.

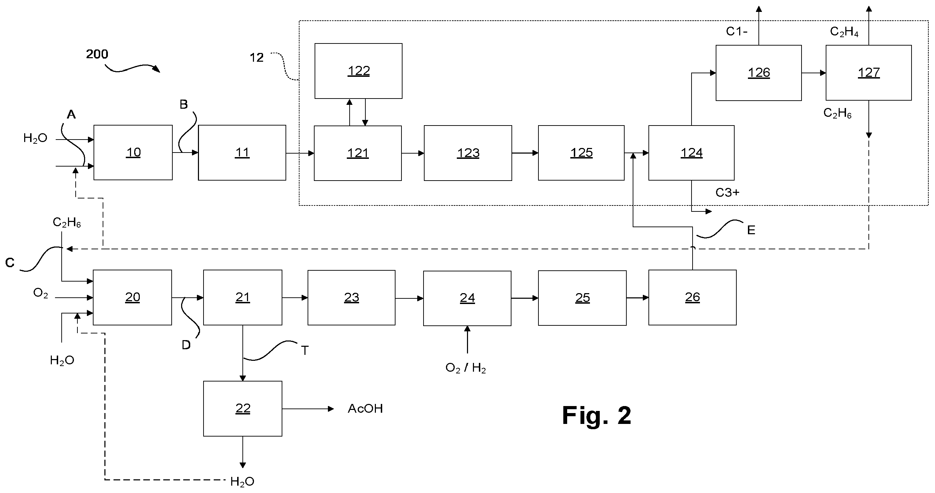

In Figur 2 ist ein Verfahren gemäß einer Ausgestaltung der Erfindung in Form eines schematischen Ablaufplans veranschaulicht und insgesamt mit 200 bezeichnet. Die Ausgestaltung 200 gemäß Figur 2 unterscheidet sich von der Ausgestaltung 100 gemäß Figur 1 im Wesentlichen durch die abweichende, hier als Rohgas-Hydrierung ausgeführte Selektiventfernung 125. Im Übrigen wird auf die obigen Ausführungen verwiesen.

Nachfolgend werden nochmals Vorteile und Ausgestaltungen der vorliegenden Erfindung unter expliziter Bezugnahme auf den Stand der Technik erläutert.

Die oxidative Dehydrierung von Alkanen, insbesondere von Ethan, kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen und der praktisch irreversiblen Wasserbildung keine thermodynamische Gleichgewichtslimitierung. Die oxidative Dehydrierung kann bei vergleichsweise geringen Reaktionstemperaturen durchgeführt werden. Grundsätzlich ist keine Regenerierung der eingesetzten Katalysatoren erforderlich, da die

Anwesenheit von Sauerstoff eine in-situ-Regenerierung ermöglicht bzw. bewirkt. Schließlich werden im Gegensatz zum Steamcracken geringere Mengen an wertlosen Nebenprodukten wie Koks gebildet. In Ausgestaltungen der vorliegenden Erfindung ist die oxidative Dehydrierung, insbesondere von Ethan, insbesondere deshalb vorteilhaft, weil sie die vorteilhafte Nutzung von Ethan ermöglicht, auch wenn das Steamcracken zur Nutzung (rein oder überwiegend) flüssiger Einsätze ausgelegt ist.

Zu weiteren Details bezüglich der oxidativen Dehydrierung sei auf Fachliteratur, beispielsweise Ivars, F. und Löpez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 12, 2013, Seiten 3196 bis 3247, sowie X. Li, E. Iglesia, Kinetics and Mechanism of Ethane Oxidation to Acetic Acid on Catalysts Based on Mo-V-Nb Oxides, J. Phys. Chem. C, 2008, 125, 15001-15008, verwiesen.

In Ausgestaltungen der vorliegenden Erfindung besonders relevante Aspekte der oxidativen Dehydrierung und weiterer technischer Hintergrund von entsprechenden Ausgestaltungen werden nachfolgend weiter erläutert.

Das Produktgas der oxidativen Dehydrierung von Ethan nach einer Kondensatabtrennung enthält signifikante Mengen an Acetylen, beispielsweise bis zu 500 vol.-ppm, 400 vol.-ppm oder 300 vol.-ppm und mehr als 10 vol.-ppm, 50 vol.-ppm, oder 100 vol.-ppm, an Kohlenmonoxid, beispielsweise bis zu 5 vol%, 4 vol%, oder 3 vol% und mehr als 0,5 vol%, oder 1 vol%, an Kohlendioxid, beispielsweise bis zu 4 vol%, 3 vol% oder 2vol% und mehr als 0,5 vol% oder 1 vol% und an Sauerstoff, beispielsweise bis zu 2 mol%, 1,5 mol%, 1 mol%, oder 0,5 mol% und mehr als 500 vol.-ppm, 1000 vol.-ppm oder 2500 vol.-ppm. Außer Acetylen sind weitere einfach und mehrfach ungesättigten Kohlenwasserstoffe (höhere Olefine, Diene und höhere Acetylene) nur im Spurenbereich im Produktgas enthalten.

Entsprechende Optionen zur Entfernung von restlichem Sauerstoff und Acetylenen, zuvor auch als "Spurenentfernung" bezeichnet, sind prinzipiell bekannt und beispielsweise in der WO 2020/187572 A1 oder auch in der WO 2018/153831 A1 offenbart. Dabei können typischerweise Restgehalte an Sauerstoff von weniger als 1000 vol.-ppm, 500 vol.-ppm, 100 vol.-ppm, 10 vol.-ppm oder 1 vol.-ppm, an Acetylen von weniger als 5 vol.-ppm, 2 vol.-ppm, 1 vol.-ppm, 0,5 vol.-ppm, 0,3 vol.-ppm oder 0,1 vol.-ppm erreicht werden. In Ausgestaltungen der vorliegenden Erfindung ist eine vollständige Entfernung von Sauerstoff und/oder Acetylen nicht notwendig. Nachfolgend wird auch der oben definierte Begriff "Rohgasbehandlung" verwendet.

Wie beispielsweise in der WO 2018/153831 A1 beschrieben, können Sauerstoff, Kohlenmonoxid und optional Acetylen aus dem Produktstrom einer oxidativen Dehydrierung von Ethan entfernt werden. Hierbei ist allgemein die Verwendung eines Oxidationskatalysators offenbart, der als Bestandteile insbesondere die Metalle Niob, Kupfer, Zink, Palladium, Silber, Platin, Gold, Eisen, Mangan, Cer, Zinn, Rubidium und Chrom aufweisen kann. Dabei werden bevorzugt kupfer- und/oder platinbasierte Systeme, insbesondere aber kupferbasierte Systeme, verwendet. Ausgestaltungen der vorliegenden Erfindung können unter Verwendung sämtlicher aus dem Stand der Technik und der Fachliteratur bekannter Acetylen- und Sauerstoffentfernungsverfahren durchgeführt werden.

Bei der oxidativen Dehydrierung, insbesondere bei Einsatz der genannten Katalysatorsysteme, entsteht Essigsäure als Koppelprodukt (typisches Verhältnis 3 bis 12 mol/mol Ethylen zu Essigsäure), der Bedarf an Essigsäure ist jedoch oftmals limitiert auf spezielle stromabwärtige Produkte bzw. Verfahren (z.B. die Herstellung von Vinylacetatmonomer, VAM). Essigsäure kann als Bestandteil der stromab der oxidativen Dehydrierung abgetrennten Kondensatphase (der typische Essigsäuregehalt im Kondensat liegt bei 1 bis 30 Gew.-%, 3 bis 25 Gew.-% oder 5 bis 20 Gew.-%) abgetrennt werden und durch eine geeignete Aufbereitung als eigenes Wertprodukt bereitgestellt werden, wie erläutert.

Andererseits entstehen im Gegensatz zum Steamcracker bei der oxidativen Dehydrierung von Ethan - abgesehen von den Nebenprodukten Kohlenmonoxid und Kohlendioxid - aber eben auch nur Ethylen und Essigsäure als Produkte. Somit entfällt auch die Anforderung einer geeigneten Verwertung weiterer Produktfraktionen aus der oxidativen Dehydrierung und der entsprechenden apparativen Einrichtungen im Zerlegungsteil. In Ausgestaltungen der vorliegenden Erfindung ist eine Nutzung entsprechender Produkte besonders vorteilhaft möglich.

Die beim Steamcracken eingesetzte Technologie ist ebenfalls umfangreich vorgeschrieben. Wichtig ist hier typischerweise insbesondere die strikte Einhaltung der Produktspezifikation von Ethylen in Hinblick auf stromabwärtige Prozesse (z.B. Polyethylenherstellung, Ethylenoxidherstellung etc.). Hier sind neben anderen insbesondere für Kohlenmonoxid, Kohlendioxid, Sauerstoff und Acetylen sehr strenge Grenzwerte einzuhalten, die durch entsprechende Reinigungen und Trennschritte erreicht werden. Besonders relevante Prozessschritte sind nachfolgend aufgeführt und näher erläutert.

In Ausgestaltungen der vorliegenden Erfindung sind insbesondere bestimmte Komponenten des Produktgemischs bzw. Rohgasstroms beim Steamcracken und Aspekte von dessen weiterer Behandlung besonders relevant. Insbesondere umfasst ein entsprechendes Produktgemisch einen hohen Anteil an mehrfach ungesättigten Kohlenwasserstoffen (Diene und Acetylene) und weist einen geringen Kohlenmonoxid- und Kohlendioxidgehalt auf. Aspekte der Behandlung umfassen die Entfernung von Kohlenmonoxid mittels Demethanisierung (siehe unten) und/oder die Entfernung von Kohlendioxid, üblicherweise mittels Laugewäsche (siehe unten).

Eine Selektivhydrierung von Kohlenwasserstoffen mit zwei Kohlenstoffatomen dient dazu, den Acetylengehalt spezifikationsgerecht zu reduzieren. Dabei wird Acetylen über geeigneten Katalysatoren selektiv zu Ethylen hydriert. Die Selektivhydrierung erfolgte früher überwiegend mit nickelbasierten Katalysatoren, heutzutage werden vornehmlich palladiumbasierte Katalysatoren mit geeigneten Dotierungen wie Silber, Gold, Cer u.a. eingesetzt. Es besteht eine beträchtliche Sensitivität gegenüber Kohlenmonoxid. Kohlenmonoxid dient als Moderator und der Kohlenmonoxidgehalt in der Hydrierung liegt typischerweise im Bereich von 50 bis 2500 vol.-ppm, 100 bis 1500 vol.-ppm oder 150 bis 1000 vol.-ppm. Diese Kohlenmonoxidgehalte liegen deutlich unterhalb der o.g. Kohlenmonoxidgehalte eines Prozessgases einer oxidativen Dehydrierung von Ethan und auch geringer als in Fällen, in denen eine Rohgasbehandlung eingesetzt wird.

Hohe Kohlenmonoxidgehalte in der Selektivhydrierung können dabei bedingt durch eine höhere Temperatur ausgeglichen werden, dies führt jedoch ggf. zu Ethylenverlust. Die maximale Temperatur ist dabei einerseits durch die Designtemperatur des Reaktors, insbesondere bei bestehenden Anlagen, aber auch durch das Betriebsfenster des Katalysators begrenzt. Wird der o.g. Temperaturbereich überschritten, lässt sich die Selektivhydrierung nicht mehr betreiben bzw. es ist der Einsatz besonders angepasster Katalysatoren erforderlich.

Grundsätzlich ist die Selektivhydrierung auch nicht unempfindlich gegenüber der Anwesenheit von Sauerstoff, da bei Anwesenheit von Sauerstoff Wasser gebildet werden kann, was wiederum die Bildung von weiteren Nebenprodukten wie Carbonsäuren und Grünöl fördert. In Ausgestaltungen der vorliegenden Erfindung werden entsprechende Probleme in besonders vorteilhafter Weise ausgeräumt.

Als Positionen für eine Selektivhydrierung sind verschiedene Varianten bekannt:
(1) Eine Rohgashydrierung ist unmittelbar nach Verdichtung, Laugewäsche und Trocknung positioniert. Diese kommt vorzugsweise in Gascrackern, also mit gasförmigen Einsätzen betriebenen Steamcrackern zur Anwendung. Dabei werden ebenfalls höhere mehrfach ungesättigte Kohlenwasserstoffe zumindest anteilig umgesetzt (insbesondere Butadien, Methylacetylen und Propadien). Typische Kohlenmonoxidgehalte liegen im unteren Bereich der o.g. Spanne, also insbesondere im Bereich von 50 bis 1000 vol.-ppm oder 50 bis 500 vol.-ppm.
(2) Eine sogenannte Frontendhydrierung ist in einer ersten Ausführungsform nach einer Deethanisierung positioniert. Hier handelt es sich also um eine reine Acetylenhydrierung und höhere Kohlenwasserstoffe als solche mit zwei Kohlenstoffatomen sind im Feedstrom der Selektivhydrierung nicht enthalten. In beiden Fällen sind neben Ethan zudem entsprechende Mengen an Methan, Wasserstoff und Kohlenmonoxid im Feedstrom der Selektivhydrierung enthalten. Typische Kohlenmonoxidgehalte liegen hier verfahrensbedingt höher als bei der Rohgashydrierung, also insbesondere im Bereich von 100 bis 2500 vol.-ppm oder 100 bis 1500 vol.-ppm. Als Variante ist auch die Anordnung nach einer Depropanisierung bekannt, in diesem Fall umfasst der Feedstrom der Hydrierung also auch Kohlenwasserstoffe mit drei Kohlenstoffatomen. Ähnlich wie bei der Rohgashydrierung werden dann die entsprechenden höheren mehrfach ungesättigten Kohlenwasserstoffe zumindest anteilig umgesetzt.
(3) Prinzipiell ist auch eine so genannte Tailendhydrierung bekannt, bei der vor der Hydrierung zumindest noch der ein Strom mit Methan und anderen leichten Komponenten abgetrennt wird und entsprechend eine stöchiometrische Wasserstoffdosierung erforderlich ist. Diese Variante ist in Ausgestaltungen der vorliegenden Erfindung jedoch weniger relevant.

In Ausgestaltungen der vorliegenden Erfindung werden insbesondere eine Rohgas- oder Frontendhydrierung eingesetzt, wie zu den Punkten (1) und (2) erläutert.

Neben der Anforderung an die hohe Produktreinheit des Ethylenproduktes machen auch kryogene Anlagenteile eine quantitative Abscheidung von Kohlendioxid notwendig, um ein Ausfrieren von Kohlendioxid und damit Verlegungen zu vermeiden.

Kohlendioxid kann - insbesondere in Gehalten, wie sie bei der oxidativen Dehydrierung von Ethan auftreten - aufgrund seiner hohen Wechselwirkung mit geeigneten Lösungsmitteln bzw. Waschflüssigkeiten ebenso vergleichsweise einfach aus dem Produktgemisch entfernt werden, wobei bekannte Verfahren zur Kohlendioxidentfernung, insbesondere entsprechende Wäschen (beispielsweise Aminwäschen) zum Einsatz kommen können. Die beladene Waschflüssigkeit wird dann in einer separaten Kolonne regeneriert und dabei wird sehr reines Kohlendioxid durch Desorption freigesetzt.

Sollten Folgeschritte die Abwesenheit oder nur eine sehr geringe Restkonzentration von Kohlendioxid erfordern (z.B. aufgrund einer Katalysatorinhibierung oder sogenannten Katalysatorvergiftung), kann der Restgehalt an Kohlendioxid nach einer Aminwäsche bedarfsweise durch eine optionale Laugewäsche als Feinreinigung anforderungsgemäß weiter reduziert werden.

Mit gewissen Ausnahmen können die entsprechenden Waschflüssigkeiten auch mit Sauerstoff reagieren, wodurch es im Laufe der Zeit zu einer nachteiligen Alterung bzw. Schädigung der Waschmittel kommen kann, die einen kontinuierlichen Purge- und Makeup-Strom erfordern bzw. zu einer unerwünschten Verkürzung der Lebensdauer dieser Waschflüssigkeiten führen. Daher ist auch aus diesem Aspekt die Entfernung von Sauerstoff stromauf einer entsprechenden Wäsche vorteilhaft.

Im Rohgasstrom eines Steamcrackers liegen typischerweise deutlich geringere Kohlendioxidgehalte als bei der oxidativen Dehydrierung vor. Diese werden üblicherweise mit einer Laugewäsche entfernt. Ein um mehrere Größenordnungen höherer Kohlendioxidgehalt wie bei der oxidativen Dehydrierung von Ethan würde wie zuvor ausgeführt zu einem übermäßigen Laugeverbrauch führen. In der vorliegenden Erfindung wird daher wie erwähnt eine separate Abscheidung von Kohlendioxid mittels Amin- und optional Laugewäsche stromab der oxidativen Dehydrierung vorgenommen.

Die Entfernung von Wasser erfolgt gemäß Stand der Technik mittels regenerativer Trockner auf Molsiebbasis und ist neben der Erreichung der Produktspezifikation ebenfalls zwingend in Hinblick auf nachfolgende kryogene Prozessschritte, um dort Verlegungen durch Abscheidung von Eis und Hydraten zu vermeiden.

Typischerweise muss im Rahmen einer geeigneten Verfahrensführung sowohl bei einer oxidativen Dehydrierung von Ethan, als auch im Zerlegungsteil eines Steamcrackers auch im Produktstrom enthaltenes Methan (z.B. insbesondere aus dem Ethaneinsatzstrom der oxidativen Dehydrierung stammend) entfernt werden. Dazu kommt üblicherweise eine Demethanisierung zum Einsatz, die entsprechende kryogene Bedingungen erfordert. Eine Demethanisierung entfernt dabei gleichzeitig Kohlenmonoxid und Wasserstoff aus dem entsprechenden Strom. Es wird also eine mit C1- (sprich "C1minus") bezeichnete Fraktion gebildet, die als wesentliche Bestandteile Methan, Wasserstoff und/oder Kohlenmonoxid enthält. Im Eintrittsstrom der Demethanisierung noch enthaltene Spuren von Sauerstoff gelangen dabei ebenfalls in diese Fraktion. Daher wird üblicherweise angestrebt, den Eintrittsgehalt an Sauerstoff im Eintrittsstrom zur Demethanisierung zu begrenzen und so die mögliche Bildung einer explosionsfähigen Atmosphäre am Kopf zu vermeiden. Eine entsprechende Auslegung des Demethanizers ist zwar möglich, wie beispielsweise in der WO 2018/082945 A1 beschrieben, bedeutet aber entsprechend deutlich erhöhten apparativen Aufwand. Die EP 3 456 703 A1 beschreibt eine Demethanisierung im Zerlegungsteil einer Anlage zur oxidativen Dehydrierung von Ethan, die mit einer Druckwechseladsorption im Kopfstrom kombiniert wird. Zur Minimierung von Produktverlusten wird des Weiteren angestrebt, den Ethylengehalt im Kopfgas des Demethanizers zu minimieren.

Schlussendlich muss nicht umgesetztes Ethan von Ethylen getrennt werden, was mittels eines mehrfach erwähnten C2-Splitters, der ebenfalls unter kryogenen Bedingungen betrieben wird, erfolgt. Dieser muss also so gebaut und betrieben werden, dass Ethan im Ethylen praktisch quantitativ entfernt wird (geforderter Schlussendlich muss nicht umgesetztes Ethan von Ethylen getrennt werden, was mittels eines C2-Splitters, der ebenfalls unter kryogenen Bedingungen betrieben wird, erfolgt. Dieser muss also so gebaut und betrieben werden, dass Ethan im Ethylen praktisch quantitativ entfernt wird (geforderter Reinheitsgrad des Ethylen-Produkts i.d.R. mehr als 99,9 %) und gleichzeitig ein Ethanstrom, der zur oxidativen Dehydrierung zurückgeführt wird, möglichst wenig bzw. kein Ethylen enthält.

Ansätze zur Integration von Steamcracker und oxidativer Dehydrierung von Ethan sind grundsätzlich bekannt, wie bereits erwähnt. Allen eingangs genannten Schriften ist gemeinsam, dass sie nicht auf die wohlbekannte Entstehung von Essigsäure als Koppelprodukt der oxidativen Dehydrierung eingehen und jeweils nur eine wässrige Kondensatphase abtrennen. Ausgestaltungen der vorliegenden Erfindung berücksichtigen dagegen insbesondere auch diesen Aspekt.

Die WO 2018/024650 A1 fokussiert insbesondere auf die Integration eines Steamcrackers mit Ethaneinsatz mit einer oxidativen Dehydrierung von Ethan. Die Einspeisung eines Prozessgases aus einer oxidativen Dehydrierung von Ethan in den Zerlegungsteil eines Steamcrackers wird grundsätzlich beschrieben und beansprucht. Es sind jedoch keine Lösungsansätze offenbart, welche die Problematik der deutlich abweichenden Sauerstoff-, Kohlenmonoxid- und Acetylengehalte im Prozessgas aus einer oxidativen Dehydrierung von Ethan und in entsprechenden Strömen im Zerlegungsteil eines Steamcrackers aufgreifen und lösen. Eine Tailendhydrierung, und eine Zusammenführung von Produktströmen stromauf dieser Tailendhydrierung ist beschrieben, so dass die Kohlenwasserstoffe mit zwei Kohlenstoffatomen aus Steamcracker und oxidativen Dehydrierung von Ethan also einer gemeinsamen Hydrierung unterzogen werden. Dies steht genau im Gegensatz zum Lösungsansatz von Ausgestaltungen der vorliegenden Erfindung. Dementsprechend ist auch keine Rohgasbehandlung des Prozessstromes einer oxidativen Dehydrierung von Ethan dargestellt oder beansprucht.

Die WO 2014/134703 A1 offenbart eine Sauerstoffentfernung unmittelbar stromab eines Reaktors zur oxidativen Dehydrierung von Ethan (sogenannter "Afterburner"), es werden typische Restsauerstoffgehalte von weniger als 1000 vol.-ppm genannt. Zwar werden in allgemeiner Weise Integrationsansätze von oxidativer Dehydrierung von Ethan und Steamcracken beansprucht, die wahlweise die Prozesseinheiten C2-Splitter und/oder eine Acetylenhydrierung umfassen. Dabei kann die Integration sowohl explizit stromauf als auch explizit stromab der Acetylenhydrierung erfolgen. Jedoch werden auch hier keine Lösungen für die Problematik der deutlich abweichenden Kohlenmonoxid- und Acetylengehalte im Prozessgas aus einer oxidativen Dehydrierung von Ethan und in entsprechenden Strömen im Zerlegungsteil eines Steamcrackers aufgezeigt. Auch die vorteilhafte Positionierung eines Demethanizers und Integration im Gesamtverfahren wird in der Schrift nicht näher betrachtet.

Die CN 103086821 B betrifft die Integration eines Naphthacrackers mit einer oxidativen Dehydrierung von Ethan. Es finden sich zwar grundsätzliche Ausführungen zur Entfernung von Sauerstoff, Kohlenmonoxid und Kohlendioxid. Jedoch offenbart die Schrift keine Lösung für die Entfernung von Acetylen. Vielmehr lehrt diese Schrift im Gegensatz zu den zuvor gemachten Ausführungen, dass bei der oxidativen Dehydrierung von Ethan von Ethan gerade keine Nebenprodukte wie Essigsäure und Acetylen entstehen sollen.

Ausgestaltungen der vorliegenden Erfindung erfüllen zuvor dargestellten gegensätzlichen Anforderungen und ermöglichen eine optimierte Integration einer oxidativen Dehydrierung von Ethan und eines Steamcrackers.

Eine Reihe von Aspekten steht nämlich der Einspeisung eines Produktgases aus der oxidativen Dehydrierung von Ethan in den Zerlegungsteil eines Steamcrackers entgegen. So ist ein hoher Kohlenmonoxidgehalt im Prozessgas der oxidativen Dehydrierung von Ethan limitierend für die Hydrierung von Kohlenwasserstoffen mit zwei Kohlenstoffatomen; hier kann zunächst nur eine so genannte Tailendhydrierung zur Anwendung kommen bzw. es können nur spezielle Katalysatoren verwendet werden, die hohe Kohlenmonoxidgehalt tolerieren. Ein zu hoher Kohlendioxidgehalt im Prozessgas der Kohlenmonoxidgehalt spricht gegen eine Entfernung mittels typischer Laugewäsche. Der Sauerstoffgehalt im Prozessgas der oxidativen Dehydrierung kann zu Foulingeffekten, einer möglichen Anreicherung von Sauerstoff in der erwähnten C1minus-Fraktion, und daher zu einer sicherheitstechnischen Gefährdung führen. Dabei bleibt der Sauerstoffgehalt auch nach einer gesonderten Rohgasbehandlung im Prozessgas der oxidativen Dehydrierung ggf. trotzdem kritisch wegen mehrfach ungesättigter Kohlenwasserstoffe aus dem Cracker bzw. es stellt sich hier eine sehr scharfe Reinheitsanforderung (weniger als 10 vol.-ppm, insbesondere weniger als 1 vol.-ppm Sauerstoff) an eine solche Rohgasbehandlung, die entsprechenden Aufwand bedingt und zu Ethylenverlusten führen kann.

Ausgestaltungen der Erfindung lösen diese Probleme durch die mehrfach erwähnte Einspeisung des Produktgasstromes aus einer oxidativen Dehydrierung nach geeigneter Vorbehandlung an geeigneter Stelle in den Zerlegungsteil eines Steamcrackers. Einerseits erfolgt dabei in Ausgestaltungen der Erfindung die Vorbehandlung des Produktgasstroms der oxidativen Dehydrierung umfassend eine separate Verdichtung und Rohgasbehandlung und ggf. Kohlendioxidentfernung (insbesondere Aminwäsche, hier wird vorteilhafterweise Kohlendioxid als eigenes reines Produkt erhalten zur möglichen weiteren Verwendung und/oder sogenannten Carbon Capture & Storage (CCS), beispielsweise in Form einer Verpressung in den Boden. Bevorzugt wird die Kohlendioxidentfernung aus dem Produktgasstrom der oxidativen Dehydrierung von Ethan zweistufig ausgeführt (Aminwäsche und Laugewäsche), um vor Zuspeisung in den Zerlegungsteil eines Steamcrackers ähnlich niedrige Kohlendioxidgehalte wie im Spaltgas des Crackers stromab der Laugewäsche zu erreichen (i.d.R. Werte geringer als 1 mol-ppm oder noch geringer). Gegebenenfalls erfolgt eine Trocknung. Andererseits erfolgt in Ausgestaltungen der Erfindung eine Einspeisung in den Zerlegungsteil eines Steamcrackers stromab einer Selektivhydrierung von Kohlenwasserstoffen mit zwei Kohlenstoffatomen (Frontend- oder Rohgas-Hydrierung) und stromauf einer Demethanisierung.

Notwendige Anpassungen der Temperaturniveaus sind hier und in den vorstehend erläuterten Figuren 1 und 2 nicht explizit berücksichtig und erfolgen auf dem Fachmann bekannte Art und Weise (z.B. Wärmetauscher).

In einer Ausgestaltung der Erfindung werden besonders bevorzugt zumindest Teile des vereinigten Produktgasstroms an einzelnen Stellen des Prozesses bis auf Temperaturen von weniger als -120°C, -135°C oder -150°C gekühlt. Das Erreichen besonders niedriger Temperaturen begünstigt die Minimierung von Ethylenverlusten in der leichten Fraktion C1-, die wie erwähnt im Demethanizer aus dem vereinigten Produktgasstrom abgetrennt wird.

Hieraus ergibt sich ein zusätzlicher Synergievorteil der Integration, da die bei einer eigenständigen, nicht integrierten Anlage zur oxidativen Dehydrierung von Ethan anteilsmäßig nur eine kleine C1minus-Fraktion zur Verfügung steht. Diese ist nicht tauglich für die Erzeugung nutzbarer Spitzenkälte und es können in einer eigenständigen Anlage zur oxidativen Dehydrierung Temperaturen unterhalb von - 110°C nicht ohne erheblichen zusätzlichen Aufwand erreicht werden. Diese Situation verbessert sich signifikant, wenn man den Produktgasstrom der oxidativen Dehydrierung von Ethan mit dem des Crackers vereinigt und einem gemeinsamen Demethanizer zuführt. So können zumindest anteilig für die Fraktion der oxidativen Dehydrierung von Ethan durch die Verfügbarkeit von Spitzenkälte im o.g. Temperaturbereich Ethylenverluste reduziert werden.

## Patentansprüche

1. Verfahren (100, 200) zur Herstellung eines oder mehrerer Kohlenwasserstoffe, bei dem ein erster Einsatzstrom (A) unter Erhalt eines ersten Produktstroms (B) einem Steamcracken (10) und ein Ethan enthaltender zweiter Einsatzstrom (C) unter Erhalt eines zweiten Produktstroms (D) einer oxidativen Dehydrierung (20) unterworfen werden, wobei zumindest ein Teil des ersten Produktstrom (B) unter Erhalt von Kohlenwasserstofffraktionen (C₂H₄, C₂H₆) einer Aufbereitung (12) unterworfen wird, die eine Selektivhydrierung (125) von Kohlenwasserstoffen mit zwei Kohlenstoffatomen und eine Demethanisierung (126) umfasst, zumindest ein Teil des zweiten Produktstroms (D) unter Erhalt eines Folgestroms (E) einer Spurenentfernung (24) unterworfen wird, die eine Entfernung von Sauerstoff und/oder Acetylen umfasst, und zumindest ein Teil des Folgestroms (E) der Aufbereitung (12) an einer Einspeisestelle stromab der Selektivhydrierung (125) und stromauf der Demethanisierung (126) zugeführt wird, wobei zumindest ein Teil des Folgestroms (E) stromauf der Einspeisestelle in die Aufbereitung (12) einer Entfernung (25) von Kohlendioxid unterworfen wird, wobei ein Restsauerstoffgehalt des Folgestroms (E) weniger als 500 vol.-ppm beträgt und wobei ein Restacetylengehalt des Folgestroms (E) weniger als 5 vol.-ppm beträgt.

2. Verfahren (100, 200) nach Anspruch 1, bei dem die Entfernung (25) von Kohlendioxid zumindest eine regenerative Wäsche, insbesondere eine Aminwäsche, umfasst.

3. Verfahren (100, 200) nach Anspruch 1 oder 2, bei dem die oxidative Dehydrierung (20) unter Verwendung eines oder mehrerer, die Metalle Molybdän, Vanadium, Niob und optional Tellur enthaltender Katalysatoren durchgeführt wird.

4. Verfahren (100, 200) nach einem der vorstehenden Ansprüche, bei dem die Aufbereitung (12) eine Deethanisierung (124) und/oder eine Depropanisierung umfasst, wobei die Selektivhydrierung (125) stromab der Deethanisierung (124) und/oder stromab der Depropanisierung vorgenommen wird.

5. Verfahren (100, 200) nach einem der Ansprüche 1 bis 3, bei dem die Aufbereitung (12) eine Deethanisierung (124) umfasst, wobei die Selektivhydrierung (125) stromauf der Deethanisierung (124) vorgenommen wird.

6. Verfahren (100, 200) nach einem der vorstehenden Ansprüche, bei dem zumindest ein Teil des Folgestroms (E) stromauf der Einspeisestelle in die Aufbereitung (12) einer Verdichtung (23) und/oder einer Trocknung (26) unterworfen wird.

7. Verfahren (100, 200) nach einem der vorstehenden Ansprüche, bei dem in der Selektivhydrierung (125) ein zumindest Palladium enthaltender Katalysator verwendet wird.

8. Verfahren (100, 200) nach einem der vorstehenden Ansprüche, bei dem in der Spurenentfernung (24) ein oder mehrere Kupferoxid enthaltende Katalysatoren oder ein oder mehrere, zumindest eines der Elemente Kupfer, Mangan, Zink, Nickel, Platin, Palladium, Rhodium und/oder Ruthenium enthaltende Katalysatoren verwendet werden.

9. Verfahren (100, 200) nach einem der vorstehenden Ansprüche, bei dem ein Restsauerstoffgehalt des Folgestroms (E) weniger als 250 vol.-ppm, 100 vol.-ppm, 10 vol.-ppm oder 1 vol.-ppm beträgt.

10. Verfahren (100, 200) nach einem der vorstehenden Ansprüche, bei dem ein Restacetylengehalt des Folgestroms (E) weniger als 2 vol.-ppm, 1 vol.-ppm, 0,5 vol.-ppm, 0,3 vol.-ppm oder 0,1 vol.-ppm beträgt.

11. Verfahren (100, 200) nach einem der vorstehenden Ansprüche, bei dem die Aufbereitung (12) eine Trennung (127) von Kohlenwasserstoffen mit zwei Kohlenstoffatomen umfasst, in der ein an Ethan angereicherter Strom (U) gewonnen wird, wobei der an Ethan angereicherte Strom (U) zumindest teilweise zum Steamcracken (10) und/oder zur oxidativen Dehydrierung (20) als Teil des ersten und/oder zweiten Einsatzstroms (A, C) zurückgeführt wird.

12. Verfahren (100, 200) nach einem der vorstehenden Ansprüche, bei die Spurenentfernung (24) mittels einer Rohgasbehandlung erfolgt, die vor oder nach einer Verdichtung angeordnet ist.

13. Verfahren (100, 200) nach einem der vorstehenden Ansprüche, bei dem in der Aufbereitung (12) stromab der Einspeisestelle eine Abkühlung zumindest eines Teils des jeweils bearbeiteten Gasgemischs auf Temperaturen von weniger als - 120°C, -135°C oder -150°C vorgenommen wird.

14. Verfahren (100, 200) nach einem der vorstehenden Ansprüche, bei dem zumindest ein Teil des zweiten Produktstroms (D) einer Abtrennung (21) von Kondensat unterworfen wird, in der ein Kondensatstrom (T) abgetrennt wird, der mindestens 1 Gew.-% Essigsäure enthält, wobei der Kondensatstrom (T) insbesondere einer weiteren Aufbereitung unterzogen wird, um Essigsäure als Wertprodukt zu gewinnen.

15. Anlage, die zur Durchführung eines Verfahrens nach einem der vorstehenden Ansprüche eingerichtet ist.

## Claims

1. A method (100, 200) for producing one or more hydrocarbons, wherein a first feed stream (A) is subjected to a steam cracking stage (10) to obtain a first product stream (B), and a second feed stream (C) containing ethane is subjected to an oxidative dehydrogenation stage (20) to obtain a second product stream (D), wherein at least a portion of the first product stream (B) undergoes a treatment stage (12) to obtain hydrocarbon fractions (C₂H₄, C₂H₆), the treatment stage comprising the selective hydrogenation stage (125) of hydrocarbons having two carbon atoms and a demethanization stage (126), at least a portion of the second product stream (D) undergoes a trace removal stage (24), which comprises the removal of oxygen and/or acetylene, to obtain an after-stream (E), and at least a portion of the after-stream (E) is fed to the treatment stage (12) at a feed point downstream of the selective hydrogenation stage (125) and upstream of the demethanization stage (126), wherein at least a portion of the after-stream (E) upstream of the feed point into the treatment stage (12) undergoes a carbon dioxide removal stage (25), wherein a residual oxygen content of the after-stream (E) is less than 500 vol. ppm and wherein a residual acetylene content of the after-stream (E) is less than 5 vol. ppm.

2. The method (100, 200) according to claim 1, wherein the removal (25) of carbon dioxide comprises at least one regenerative scrubbing stage, in particular an amine scrubbing stage.

3. The method (100, 200) according to claim 1 or 2, wherein the oxidative dehydrogenation (20) is carried out using one or more catalysts containing the metals molybdenum, vanadium, niobium and optionally tellurium.

4. The method (100, 200) according to one of the preceding claims, wherein the treatment stage (12) comprises a deethanization stage (124) and/or a depropanization stage, wherein the selective hydrogenation stage (125) is carried out downstream of the deethanization stage (124) and/or downstream of the depropanization stage.

5. The method (100, 200) according to one of claims 1 to 3, wherein the treatment stage (12) comprises a deethanization stage (124), wherein the selective hydrogenation stage (125) is carried out upstream of the deethanization stage (124).

6. The method (100, 200) according to one of the preceding claims, wherein at least a portion of the after-stream (E) is subjected to compression (23) and/or drying (26) upstream of the feed point into the treatment stage (12).

7. The method (100, 200) according to one of the preceding claims, wherein a catalyst containing at least palladium is used in the selective hydrogenation stage (125).

8. The method (100, 200) according to one of the preceding claims, wherein one or more catalysts containing copper oxide or one or more catalysts containing at least one of the elements copper, manganese, zinc, nickel, platinum, palladium, rhodium and/or ruthenium are used in the trace removal stage (24).

9. The method (100, 200) according to one of the preceding claims, wherein a residual oxygen content of the after-stream (E) is less than 250 vol. ppm, 100 vol. ppm, 10 vol. ppm or 1 vol. ppm.

10. The method (100, 200) according to one of the preceding claims, wherein a residual acetylene content of the after-stream (E) is less than 2 vol. ppm, 1 vol. ppm, 0.5 vol. ppm, 0.3 vol. ppm or 0.1 vol. ppm.

11. The method (100, 200) according to one of the preceding claims, wherein the treatment stage (12) comprises a separation stage (127) of hydrocarbons having two carbon atoms in which an ethane-enriched stream (U) is obtained, wherein the ethane-enriched stream (U) is at least partially fed back to the steam cracking stage (10) and/or oxidative dehydrogenation stage (20) as part of the first and/or second feed stream (A, C).

12. The method (100, 200) according to one of the preceding claims, wherein the trace removal stage (24) is carried out by means of a raw gas treatment which is arranged upstream or downstream of a compression stage.

13. The method (100, 200) according to one of the preceding claims, wherein, in the treatment stage (12) downstream of the feed point, at least a portion of the gas mixture being processed in each case is cooled to temperatures below 120 °C, -135 °C or -150 °C.

14. The method (100, 200) according to one of the preceding claims, wherein at least a portion of the second product stream (D) is subjected to a separation stage (21) of condensate, in which a condensate stream (T) is separated, which contains at least 1 wt.% acetic acid, wherein the condensate stream (T) is in particular subjected to further treatment in order to obtain acetic acid as a valuable product.

15. A system configured to carry a method according to any of the preceding claims.

## Revendications

1. Procédé (100, 200) de fabrication d'un ou plusieurs hydrocarbures, dans lequel un premier flux d'alimentation (A) est soumis à un vapocraquage (10) pour obtenir un premier flux produit (B) et un second flux d'alimentation (C) contenant de l'éthane est soumis à une déshydrogénation oxydante (20) pour obtenir un second flux produit (D), dans lequel au moins une partie du premier flux produit (B) est soumise à un traitement (12) comprenant une hydrogénation sélective (125) d'hydrocarbures à deux atomes de carbone et une déméthanisation (126) pour obtenir des fractions d'hydrocarbures (C₂H₄, C₂H₆), au moins une partie du second flux produit (D) est soumise à une élimination de traces (24) comprenant une élimination d'oxygène et/ou d'acétylène pour obtenir un flux de suite (E), et au moins une partie du flux de suite (E) du traitement (12) est dirigée vers un point d'injection en aval de l'hydrogénation sélective (125) et en amont de la déméthanisation (126), dans lequel au moins une partie du flux de suite (E) est soumise à une élimination (25) de dioxyde de carbone en amont du point d'injection dans le traitement (12), dans lequel une teneur en oxygène résiduel du flux de suite (E) est inférieure à 500 ppm en volume et dans lequel une teneur en acétylène résiduel du flux de suite (E) est inférieure à 5 ppm en volume.

2. Procédé (100, 200) selon la revendication 1, dans lequel l'élimination (25) du dioxyde de carbone comprend au moins un lavage régénératif, en particulier un lavage aux amines.

3. Procédé (100, 200) selon la revendication 1 ou 2, dans lequel la déshydrogénation oxydante (20) est mise en œuvre à l'aide d'un ou plusieurs catalyseurs contenant les métaux molybdène, vanadium, niobium et éventuellement tellure.

4. Procédé (100, 200) selon l'une des revendications précédentes, dans lequel le traitement (12) comprend une déséthanisation (124) et/ou une dépropanisation, dans lequel l'hydrogénation sélective (125) est réalisée en aval de la déséthanisation (124) et/ou en aval de la dépropanisation.

5. Procédé (100, 200) selon l'une des revendications 1 à 3, dans lequel le traitement (12) comprend une déséthanisation (124), dans lequel l'hydrogénation sélective (125) est réalisée en amont de la déséthanisation (124).

6. Procédé (100, 200) selon l'une des revendications précédentes, dans lequel au moins une partie du flux de suite (E) est soumise à une compression (23) et/ou à un séchage (26) en amont du point d'injection dans le traitement (12).

7. Procédé (100, 200) selon l'une des revendications précédentes, dans lequel un catalyseur contenant au moins du palladium est utilisé dans l'hydrogénation sélective (125).

8. Procédé (100, 200) selon l'une des revendications précédentes, dans lequel un ou plusieurs catalyseurs contenant de l'oxyde de cuivre ou un ou plusieurs catalyseurs contenant au moins un des éléments cuivre, manganèse, zinc, nickel, platine, palladium, rhodium et/ou ruthénium sont utilisés dans l'élimination de traces (24).

9. Procédé (100, 200) selon l'une des revendications précédentes, dans lequel une teneur en oxygène résiduel du flux de suite (E) est inférieure à 250 ppm en volume, 100 ppm en volume, 10 ppm en volume ou 1 ppm en volume.

10. Procédé (100, 200) selon l'une des revendications précédentes, dans lequel une teneur en acétylène résiduel du flux de suite (E) est inférieure à 2 ppm en volume, 1 ppm en volume, 0,5 ppm en volume, 0,3 ppm en volume ou 0,1 ppm en volume.

11. Procédé (100, 200) selon l'une des revendications précédentes, dans lequel le traitement (12) comprend une séparation (127) d'hydrocarbures à deux atomes de carbone dans laquelle un flux enrichi en éthane (U) est récupéré, dans lequel le flux enrichi en éthane (U) est au moins partiellement redirigé vers le vapocraquage (10) et/ou la déshydrogénation oxydante (20) en tant que partie du premier et/ou du second flux d'alimentation (A, C).

12. Procédé (100, 200) selon l'une des revendications précédentes, dans lequel l'élimination de traces (24) est effectuée au moyen d'un traitement de gaz brut qui est placé avant ou après une compression.

13. Procédé (100, 200) selon l'une des revendications précédentes, dans lequel un refroidissement d'au moins une partie du mélange gazeux respectivement traité est réalisé en aval du point d'injection dans le traitement (12) à des températures inférieures à -120 °C, -135 °C ou -150 °C.

14. Procédé (100, 200) selon l'une des revendications précédentes, dans lequel au moins une partie du second flux produit (D) est soumise à une séparation (21) de condensat dans laquelle un flux de condensat (T) contenant au moins 1 % en poids d'acide acétique est séparé, dans lequel le flux de condensat (T) est en particulier assujetti à un traitement supplémentaire afin de récupérer l'acide acétique comme produit de valeur.

15. Système configuré pour la mise en œuvre d'un procédé selon l'une des revendications précédentes.
